(19) Europäisches Patentamt
European Patent Office
Office européen des brevets

(11) EP 3 348 252 B1

# (12) EUROPEAN PATENT SPECIFICATION

(45) Date of publication and mention of the grant of the patent:
**09.06.2021 Bulletin 2021/23**

(21) Application number: **16844338.0**

(22) Date of filing: **06.09.2016**

(51) Int Cl.:
***A61K 8/29*** *(2006.01)* ***A61K 8/06*** *(2006.01)*
***A61K 8/27*** *(2006.01)* ***A61K 8/31*** *(2006.01)*
***A61K 8/86*** *(2006.01)* ***A61K 8/87*** *(2006.01)*
***A61Q 17/04*** *(2006.01)*

(86) International application number:
**PCT/JP2016/076148**

(87) International publication number:
**WO 2017/043477 (16.03.2017 Gazette 2017/11)**

## (54) OIL-IN-WATER TYPE COSMETIC AND SUNSCREEN COSMETIC

ÖL-IN-WASSER-KOSMETIKUM UND SONNENSCHUTZKOSMETIKUM

COSMÉTIQUE DE TYPE HUILE-DANS-EAU ET COSMÉTIQUE DE PROTECTION SOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2015 JP 2015177426**

(43) Date of publication of application:
**18.07.2018 Bulletin 2018/29**

(73) Proprietor: **Sumitomo Seika Chemicals Co., Ltd.**
**Kako-gun Hyogo 675-0145 (JP)**

(72) Inventors:
- **NAKATSUKA, Akio**
**Himeji-shi**
**Hyogo 672-8076 (JP)**
- **HAKOSHIMA, Yuka**
**Himeji-shi**
**Hyogo 672-8076 (JP)**

(74) Representative: **Vossius & Partner**
**Patentanwälte Rechtsanwälte mbB**
**Siebertstrasse 3**
**81675 München (DE)**

(56) References cited:
**EP-A1- 1 013 264**
**WO-A1-2014/007366**
**JP-A- 2000 072 623**
**JP-A- 2000 303 056**
**JP-A- 2005 272 389**
**JP-A- 2014 073 973**
**JP-A- 2015 155 519**
**US-A1- 2004 028 742**
**US-A1- 2012 021 027**
**US-A1- 2012 251 603**
**US-A1- 2015 183 920**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 348 252 B1

## Description

### Technical Field

The present invention relates to an oil-in-water type cosmetic and a sunscreen cosmetic comprising the oil-in-water type cosmetic.

### Background Art

Cosmetics used for cosmetic items and toiletries comprise a thickening agent, a dispersant, an emulsion stabilizer, etc. Further, for example, sunscreen cosmetics contain other additives, such as an ultraviolet scattering agent that reflects and scatters ultraviolet rays to protect the skin.

It has been proposed to formulate a sunscreen cosmetic by incorporating a UVA (320 to 400-nm) scattering agent, such as fine particulate titanium oxide or fine particulate zinc oxide, and a UVB (280 to 320-nm) scattering agent, such as a benzophenone compound, a p-aminobenzoic acid compound, or a cinnamic acid compound (see, for example, Patent Literature (PTL) 1). It has also been proposed to formulate a sunscreen cosmetic by incorporating a UVA absorber, such as 4-tert-butyl-4'-methoxydibenzoylmethane or diethylaminohydroxybenzoyl hexyl benzoate, and a UVB absorber, such as a benzophenone compound, a p-aminobenzoic acid compound, or a cinnamic acid compound (see, for example, PTL 2). Further, in recent years, in view of allergy problems caused by organic ultraviolet absorbers, a so-called nonchemical sunscreen cosmetic that contains zinc oxide and/or titanium oxide and that does not contain any organic ultraviolet absorbers has also been proposed. Such a nonchemical sunscreen cosmetic comprises carboxymethyl cellulose as a stabilizer and a (dimethylacrylamide/acryloyl dimethyltaurine sodium) crosspolymer as a thickening agent (see, for example, PTL 3. In this context the document US2012251603 is relevant.

### Citation List

### Patent Literature

PTL 1: JP2002-326906A
PTL 2: JP2011-046670A
PTL 3: JP2013-091625A

### Summary of Invention

### Technical Problem

Since ultraviolet scattering agents, such as zinc oxide, are difficult to disperse in cosmetics, such ultraviolet scattering agents have a problem that ultraviolet scattering agents may aggregate immediately or with the lapse of time, even when a compound usually used as a dispersion stabilizer, such as a carboxyvinyl polymer or an alkyl modified carboxy polymer, is added. When an ultraviolet scattering agent has poor dispersion stability, there arises, for example, a problem that an ultraviolet scattering effect, i.e., a skin-protecting effect, is not fully provided. On the other hand, for example, the above Patent Literature (PTL) 1, Patent Literature (PTL) 3, etc., disclose using a polyacrylic acid amide or a (dimethylacrylamide/sodium acryloyldimethyltaurate) crosspolymer as a thickening agent for O/W type emulsion cosmetics containing zinc oxide and titanium oxide. When such a polyacrylamide polymer is used, obtaining a water-rich product with a refreshing feel is difficult and there arises a problem in terms of the feel in usage.

The present invention was made to solve the above problem. An object of the present invention is to provide an oil-in-water type cosmetic that is excellent in the dispersion stability of an ultraviolet scattering agent, that can provide a refreshing feel and light sense of use with no stickiness to the skin, and that also has a high ultraviolet shielding effect; and a sunscreen cosmetic comprising the oil-in-water type cosmetic.

### Solution to Problem

The present inventors conducted extensive research. As a result, the inventors found that the above object can be achieved by incorporating a compound having a specific structure. The present invention has been accomplished, based on this finding.

More specifically, the present invention provides the following.

Item 1. An oil-in-water type cosmetic comprising:

(A) an ultraviolet scattering agent;
(B) a nonionic surfactant;
(C) a modified polyalkylene oxide represented by the following formula (I):

(I)

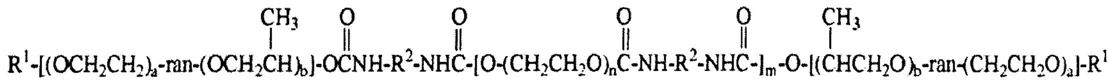

(wherein $R^1$ is a linear alkyl group having 15 to 24 carbon atoms, $R^2$ is a divalent organic residue derived from a diisocyanate compound, $[(OCH_2CH_2)_a\text{-ran-}(OCH_2CH(CH_3))_b]$ is a random copolymer of $OCH_2CH_2$ and $OCH_2CH(CH_3)$, which are constituent units of the copolymer, a is an integer of 5 to 10, b is an integer of 5 to 8, n is an integer of 130 to 680, and m is an integer of 1 to 4),
(D) an oily component, and
(E) water,
the ultraviolet scattering agent comprising fine particulate titanium oxide having a mean particle diameter of 10 to 50 nm and/or fine particulate zinc oxide having a mean particle diameter of 10 to 50 nm. Item 2. The oil-in-water type cosmetic according to Item 1, wherein the ultraviolet scattering agent is titanium oxide and/or zinc oxide.

Item 3. A sunscreen cosmetic comprising the oil-in-water type cosmetic according to Item 1 or 2.

Advantageous Effects of Invention

The oil-in-water type cosmetic of the present invention is excellent in the dispersion stability of an ultraviolet scattering agent contained in the oil-in-water type cosmetic, can provide a refreshing feel and light sense of use with no stickiness to the skin, and also has a high ultraviolet-shielding effect.
The sunscreen cosmetic of the present invention, which contains the oil-in-water type cosmetic, can provide a refreshing feel and light sense of use with no stickiness to the skin, and also has a high ultraviolet-shielding effect.

Description of Embodiments

The oil-in-water type cosmetic of the present invention comprises:

(A) an ultraviolet scattering agent;
(B) a nonionic surfactant;
(C) a modified polyalkylene oxide represented by the following formula (I):

(I)

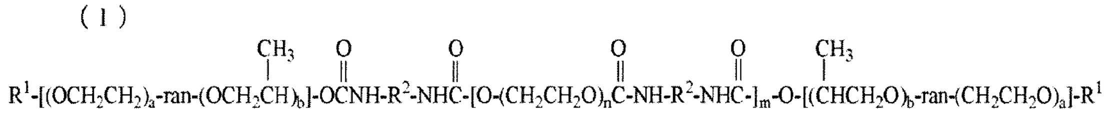

(wherein $R^1$ is a linear alkyl group having 15 to 24 carbon atoms, $R^2$ is a divalent organic residue derived from a diisocyanate compound, $[(OCH_2CH_2)_a\text{-ran-}(OCH_2CH(CH_3))_b]$ is a random copolymer of $OCH_2CH_2$ and $OCH_2CH(CH_3)$, which are constituent units of the copolymer, a is an integer of 5 to 10, b is an integer of 5 to 8, n is an integer of 130 to 680, and m is an integer of 1 to 4);
(D) is an oily component; and
(E) is water.

The ultraviolet scattering agent (A) is a material that has a property of reflecting and scattering ultraviolet rays so as to protect the skin from ultraviolet rays.
Such ultraviolet scattering agents can be used singly or in a combination of two or more. The ultraviolet scattering agent comprises fine particulate titanium oxide having a mean particle diameter of 10 to 50 nm and/or fine particulate zinc oxide having a mean particle diameter of 10 to 50 nm. In this case, the ultraviolet scattering effect of the oil-in-water type cosmetic can be further enhanced.

The ultraviolet scattering agent may be hydrophobicized. The method for the hydrophobicization treatment is not particularly limited. The hydrophobicization treatment may be performed, for example, by a usual surface-treatment method. Examples of such surface treatment methods include siliconization treatment using hydrogene dimeticone, triethoxycaprylsilane, or the like. Such methods may be used singly or in a combination of two or more.

The content of the ultraviolet scattering agent can be 0.1 to 20 mass%, and preferably 1 to 15 mass%, based on the total amount of the oil-in-water type cosmetic. A content of 0.1 mass% or more can more effectively enhance an ultraviolet-shielding effect. On the other hand, a content of 20 mass% or less is less likely to reduce the stability of the oil-in-water type cosmetic (e.g., dispersion stability).

The nonionic surfactant (B) is a component that is incorporated as an emulsifier to enhance, for example, the dispersion stability and storage stability of an oil-in-water type cosmetic. Such a nonionic surfactant is not particularly limited, and may be, for example, a lipophilic nonionic surfactant or a hydrophilic nonionic surfactant.

Examples of usable lipophilic nonionic surfactants include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerol or polyglycerol fatty acids esters, such as mono-cotton seed oil fatty acid glycerol ester, glycerol monoerucate, glycerol sesquioleate, glycerol monostearate, glycerol $\alpha,\alpha'$-oleate pyroglutamate, and glycerol monostearate maleate; propylene glycol fatty acid esters, such as propylene glycol monostearate; hydrogenated castor oil derivative; glycerol alkyl ethers; and the like.

Examples of usable hydrophilic nonionic surfactants include polyoxyethylene (POE)-sorbitan fatty acid esters, such as POE-sorbitan monooleate, POE-sorbitan monostearate, and POE-sorbitan tetraoleate; and POE-sorbitol fatty acid esters, such as POE-sorbitol monolaurate, POE-sorbitol monooleate, POE-sorbitol pentaoleate, and POE-sorbitol monostearate; POE-glycerol fatty acid esters, such as POE-glycerol monostearate, POE-glycerol monoisostearate, POE-glycerol triisostearate, and POE-glycerol monooleate; POE-fatty acid esters, such as POE-distearate, POE-monodioleate, and ethylene glycol distearate; POE-alkyl ethers, such as POE-lauryl ether, POE-oleyl ether, POE-stearyl ether, POE-behenyl ether, POE-2-octyldodecyl ether, and POE-cholestanol ether; Pluronic type surfactants, such as Pluronic L, Pluronic P, and Pluronic F, produced by Adeka Corporation; POE/POP (polyoxypropylene)-alkyl ethers, such as POE and/or POP-cetyl ether, POE/POP-2-decyltetradecyl ether, POE/POP-monobutyl ethers, POE/POP-hydrogenated lanolin, and POE/POP-glycerol ether; tetra POE/tetra POP-ethylenediamine condensates, such as Tetronic produced by BASF; POE-castor oil/POE-hydrogenated castor oil derivatives, such as POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated castor oil maleate; POE beeswax/lanolin derivatives, such as POE sorbitol beeswax; alkanolamides, such as coconut oil fatty acid diethanolamide, lauric acid monoethanolamide, and fatty acid isopropanolamides; POE-propylene glycol fatty acid esters; POE-alkylamines, POE-fatty acid amides; sucrose fatty acid esters; alkylethoxydimethylamine oxides; trioleyl phosphate; and the like.

Such nonionic surfactants may be used singly or in a combination of two or more.

When the nonionic surfactant content is 0.5 to 5 mass%, based on the total amount of the oil-in-water type cosmetic, a cosmetic comprising the oil-in-water type cosmetic can be easily applied to the skin uniformly. The nonionic surfactant content is preferably 1 to 3 mass%, based on the total amount of the oil-in-water type cosmetic.

The modified polyalkylene oxide, which is a compound (C) represented by formula (I), can be obtained, for example, by reacting a specific polyalkylene oxide compound (X), a specific polyether monohydric alcohol (Y), and a specific diisocyanate compound (Z). These components may sometimes be referred to as (X), (Y), and (Z) hereinafter.

(X) may be, for example, a polyethylene oxide represented by the following formula (II):

$$HO\text{-}(CH_2CH_2O)_n\text{-}H \qquad (II)$$

(wherein n is an integer of 130 to 680).

(X) preferably has a number average molecular weight of about 6,000 to 30,000, and more preferably about 11,000 to 20,000. When (X) has a number average molecular weight of 6,000 or more, the obtained modified polyalkylene oxide is less likely to have poor transparency. When the component (X) has a number average molecular weight of 30,000 or less, an aqueous solution of the obtained modified polyalkylene oxide is less likely to have reduced viscosity.

The number average molecular weight of the polyethylene oxide (X) is determined from the following formula using the hydroxyl number $O_1$ determined in accordance with method A in JIS K 1557-1: 2007 (Plastics - Polyols for Use in the Production of Polyurethane - Part 1: Determination of Hydroxyl Number). Specifically, the number average molecular weight is determined from the following formula, assuming that the polyethylene oxide has a hydroxyl group on each end.

$$\text{Number average molecular weight} = (56{,}100 \times 2)/O_1$$

(Y) may be, for example, a polyether monohydric alcohol having a structure in which a random copolymer of ethylene oxide (EO) and propylene oxide (PO) has a hydroxyl group at one end and a $C_{15-24}$ linear alkyl group at the other end (this alkyl group may sometimes be referred to as $R^1$ hereinafter).

(Y) may also be paraphrased as "polyoxyethylene polyoxypropylene monoalkyl ether in which ethylene oxide and propylene oxide are randomly copolymerized at a molar ratio (a/b) in the range of 1 to 2 (preferably 1.1 to 1.5) and a $C_{15-24}$ linear alkyl group is present."

(Y) can be represented, for example, by the following formula (III):

$$R^1\text{-}[(OCH_2CH_2)_a\text{-ran-}(OCH_2CH(CH_3))_b]\text{-}OH \qquad \text{(III).}$$

In formula (III), $R^1$ is a linear alkyl group having 15 to 24 carbon atoms, and $[(OCH_2CH_2)a\text{-ran-}(OCH_2CH(CH_3))_b]$ indicates that $OCH_2CH_2$ and $OCH_2CH(CH_3)$, which are constituent units, are randomly copolymerized.

As stated above, $R^1$ is a linear alkyl group having 15 to 24 carbon atoms, preferably a linear alkyl group having 16 to 22 carbon atoms, and more preferably a linear alkyl group having 16 to 20 carbon atoms. When the number of carbon atoms is 15 or more, an aqueous solution of the obtained modified polyalkylene oxide is less likely to have reduced viscosity. When the number of carbon atoms is 24 or less, an aqueous solution of the obtained modified polyalkylene oxide tends to have higher transparency.

The a/b ratio in (Y) may be, as described above, in the range of 1 to 2, and preferably 1.1 to 1.5. When the a/b ratio is 1 or more, the obtained modified polyalkylene oxide is less likely to have reduced solubility in water, and an aqueous solution of the obtained modified polyalkylene oxide is less likely to have reduced viscosity.

Preferably, a is an integer of 5 to 10. Preferably, b is an integer of 5 to 8. When a is 5 or more, an aqueous solution of the obtained modified polyalkylene oxide can have higher viscosity. When a is 10 or less, an aqueous solution of the obtained modified polyalkylene oxide can have higher transparency. When b is 5 or more, an aqueous solution of the obtained modified polyalkylene oxide can have higher transparency. When b is 8 or less, an aqueous solution of the obtained modified polyalkylene oxide can have higher viscosity.

(Y) has a structure such that a random copolymer of ethylene oxide and propylene oxide (polyalkylene oxide) is added to a linear monohydric saturated alcohol.

When a copolymer of ethylene oxide and propylene oxide is a random copolymer, the obtained modified polyalkylene oxide has excellent solubility in an aqueous medium, compared with a block copolymer, and an aqueous solution of the obtained modified polyalkylene oxide also has excellent transparency. From these viewpoints, the copolymer of ethylene oxide and propylene oxide is preferably a random copolymer.

(Y) preferably has a number average molecular weight of 800 to 3,000, and preferably 1,000 to 2,500. When the number average molecular weight is 800 or more, an aqueous solution of the obtained modified polyalkylene oxide tends to have higher viscosity. When the number average molecular weight is 3,000 or less, an aqueous solution of the obtained modified polyalkylene oxide is less likely to have reduced viscosity even in the presence of a surfactant.

The number average molecular weight of (Y) can be determined from the following formula using the hydroxy number $O_1$ determined in accordance with method A in JIS K 1557-1: 2007 (Plastics - Polyols for Use in the Production of Polyurethane - Part 1: Determination of Hydroxyl Number). Specifically, the number average molecular weight is determined from the following formula assuming that the polyether monohydric alcohol has a hydroxyl group at one end.

$$\text{Number Average Molecular Weight} = (56{,}100)/O_1$$

(Y) is preferably used in a proportion of 1 to 2 moles, more preferably 1.1 to 2 moles, and still more preferably 1.5 to 2 moles, per mole of (X). When the amount of (Y) used is 1 mole or more per mole of (X), an aqueous solution of the obtained modified polyalkylene oxide is less likely to have reduced viscosity. When the amount of (Y) used is 2 moles or less per mole of (X), the transparency of an aqueous solution of the obtained modified polyalkylene oxide tends to be more easily enhanced.

(Z) is a diisocyanate compound represented by formula (IV) :

$$O{=}C{=}N\text{-}R^2\text{-}N{=}C{=}O \qquad \text{(IV)}$$

wherein $R^2$ is a divalent organic residue derived from a diisocyanate compound, i.e., a group resulting from removal of two -N=C=O groups from a diisocyanate compound.

Specific examples of (Z) include 4,4'-diphenylmethane diisocyanate (MDI), 1,6-hexamethylene diisocyanate

(HDI), dicyclohexylmethane-4,4'-diisocyanate (HMDI), 3-isocyanatemethyl-3,5,5-trimethylcyclohexylisocyanate (IPDI), 2,6-dimethylbenzene-1,4-diisocyanate, 2,4- or 2,6-tolylene diisocyanate (TDI), and the like. From the viewpoint of excellent transparency of the obtained modified polyalkylene oxide, dicyclohexylmethane 4,4'-diisocyanate (HMDI) and 1,6-hexamethylene diisocyanate (HDI) are preferable among these diisocyanate compounds. Such diisocyanate compounds may be used singly or in a combination of two or more.

The diisocyanate compound is preferably used in the following proportion: when the total amount of the terminal hydroxyl groups in (X) and (Y) (molar amount of [-OH]) is taken as a mole, the molar amount of isocyanate groups in the isocyanate compound (molar amount of [-NCO]) is preferably 0.8 to 1.2, and more preferably 0.9 to 1.1. In other words, the isocyanate groups of the diisocyanate compound are preferably present in an amount of 0.8 to 1.2 moles, and more preferably 0.9 to 1.1 moles, when the total amount of the terminal hydroxyl groups in (X) and (Y) is taken as a mole. When the isocyanate groups are present in an amount of 0.8 moles or more, not much unreacted polyether monohydric alcohol tends to remain, and an aqueous solution of the obtained modified polyalkylene oxide is less likely to have reduced viscosity. When the isocyanate groups are present in an amount of 1.2 moles or less, the obtained modified polyalkylene oxide tends to have higher solubility in water.

Known methods can be used as a method for reacting a polyalkylene oxide compound (X) with a polyether monohydric alcohol (Y) and a diisocyanate compound (Z). Examples of such methods include a method comprising the step of dissolving or dispersing (X), (Y), and (Z) in a reaction solvent, such as toluene, xylene, or dimethylformamide, to allow a reaction to proceed; a method comprising the steps of uniformly mixing powder or solid starting materials and then heating to a predetermined temperature to allow a reaction to proceed; and the like. In view of industrial implementation, a preferable method comprises the steps of continuously supplying starting materials in a molten state to a multi-screw extruder, mixing the starting materials, and allowing a reaction to proceed in the extruder. In this case, the reaction temperature is preferably 70 to 210°C.

In the production of a modified polyalkylene oxide, a catalyst may be added to the reaction system to promote the reaction. Examples of such catalysts include triethylamine, triethanolamine, dibutyltin dilaurate, dioctyltin dilaurate, 2-ethylhexanoic acid tin, triethylenediamine, and the like.

The catalyst is preferably used in an amount of 200 to 2,000 mass ppm, and more preferably 500 to 1,000 mass ppm, based on the polyalkylene oxide compound (X).

The content of the modified polyalkylene oxide can be 0.1 to 2 mass%, based on the total amount of the oil-in-water type cosmetic. Such a content can impart a high thickening effect to the oil-in-water type cosmetic and also enhance the dispersion stability of an ultraviolet scattering agent. When a thickening agent is incorporated into an oil-in-water type cosmetic in an amount of 1 mass% or more, based on the total amount of the cosmetic, stickiness and kinking generally occur. However, from the viewpoint of obtaining the desired viscosity, the oil-in-water type cosmetic of the present invention preferably contains a modified polyalkylene oxide in an amount of 0.5 to 2 mass%.

When the oil-in-water type cosmetic is used in the form of a cream, the content of the modified polyalkylene oxide may be 0.2 to 2 mass%, based on the total amount of the oil-in-water type cosmetic. When the oil-in-water type cosmetic is used in the form of an emulsion, the content of the modified polyalkylene oxide may be 0.1 to 0.3 mass%, based on the total amount of the oil-in-water type cosmetic.

The oily component (D) is a component incorporated in the oil-in-water type cosmetic to impart, for example, texture, comfortable fitting, appearance, and smoothness to the skin.

Although the oily component is not particularly limited, examples of oily components include natural oils, silicone oils, ester oils, and the like, in view of, for example, imparting texture, comfortable fitting, appearance, and smoothness to the skin.

Examples of natural oils include paraffin hydrocarbons, such as liquid paraffin, olive oil, macadamia nut oil, castor oil, jojoba oil, orange roughy oil, beeswax, lanolin, mineral oil, and squalane.

Examples of silicone oils include modified silicone oils, such as methyl polysiloxane, methylphenyl polysiloxane, cyclic methyl siloxane, and silicone polyether copolymers.

Examples of ester oils include various fatty acid esters. Among these, octyldodecyl esters of various fatty acids, such as oleic acid, erucic acid, myristic acid, and ricinoleic acid, are preferable.

Such oily components may be used singly or in a combination of two or more.

The oily component content can be 5 to 30 mass%, based on the total amount of the oil-in-water type cosmetic.

The type of water (E) is not particularly limited. For example, distilled water, purified water, ion-exchanged water, electrolyzed water, or the like, can be used.

The water content can be 50 to 75 mass%, based on the total amount of the oil-in-water type cosmetic.

The oil-in-water type cosmetic of the present invention may comprise components other than the above components (A) to (E). Examples of other components include ultraviolet absorbers, flavoring agents, pH adjusting agents, moisturizers, solvents, preservatives, and the like. For example, solvents include ethanol and polyhydric alcohols.

The oil-in-water type cosmetic of the present invention may be in the form of, for example, an emulsion, a serum, a cream, a cream pack, a gel cream, etc. The oil-in-water type cosmetic in any of the above forms can be used

as a foundation, a sunscreen cosmetic, etc., and can be particularly suitable for use as a sunscreen cosmetic. When the oil-in-water type cosmetic is in the form of a cream, an emulsion, a gel cream, or an emulsion cosmetic, a refreshing and light feel can be provided upon application to the skin.

In the oil-in-water type cosmetic of the present invention, a modified polyalkylene oxide not only functions as a rheology control agent but also functions as a dispersant of an ultraviolet scattering agent and as an emulsion stabilizer. From this viewpoint as well, the oil-in-water type cosmetic of the present invention is particularly suitable for use as cosmetics, such as a cream, an emulsion, a gel cream, or an emulsion cosmetic, as mentioned above.

Further, the oil-in-water type cosmetic is excellent in the emulsion stability of an ultraviolet scattering agent and thereby can fully exhibit an ultraviolet scattering effect, i.e., a skin-protecting effect.

The method for preparing the oil-in-water type cosmetic of the present invention is not particularly limited. The oil-in-water type cosmetic can be prepared, for example, by a method comprising heating a container containing aqueous phase and oil phase components to about 60 to 70°C, then emulsifying the resulting solution using an emulsifying dispersion machine, such as a homomixer, followed by cooling with stirring. In this case, a nonionic surfactant (B), modified polyalkylene oxide (C), and water (E) are components constituting an aqueous phase, whereas an ultraviolet scattering agent (A) and an oily component (D) are components constituting an oily phase.

The method for preparing an oil-in-water type cosmetic is not limited to the methods described above. Alternatively, predetermined amounts of components (A) to (E) may be mixed optionally with other components using an appropriate method to prepare an oil-in-water type cosmetic.

The oil-in-water type cosmetic of the present invention is suitable for use as a starting material for producing a water-rich cosmetic with a refreshing feel. The oil-in-water type cosmetic of the present invention can be particularly suitable for use as a sunscreen cosmetic.

The method for preparing a cosmetic, such as a sunscreen cosmetic, from the oil-in-water type cosmetic of the present invention is not particularly limited. For example, known means can be used. Components generally contained in cosmetics, such as sunscreen cosmetics, may also be appropriately added. Examples

Synthesis of modified polyalkylene oxide (C)

Production Example 1

In a storage tank A equipped with a stirrer and maintained at 80°C, 100 parts by mass of fully dehydrated polyethylene oxide having a number average molecular weight of 20,000, 10 parts by mass of polyoxyethylene polyoxypropylene stearyl ether (EO/PO = 50/50, Mw = 1000), and 0.2 parts by mass of dioctyltin dilaurate were placed and stirred in a nitrogen gas atmosphere to obtain a uniform mixture. Apart from this, dicyclohexyl methane-4,4'-diisocyanate was placed in a storage tank B maintained at 30°C and stored in a nitrogen gas atmosphere.

Using metering pumps, the mixture in the storage tank A and dicyclohexyl methane-4,4'-diisocyanate in a storage tank B were continuously fed at respective rates of 500 g/min and 11.9 g/min into a twin screw extruder set at 110 to 140°C (R value = 1.00), and mixed in the extruder to allow a reaction to proceed. A strand was then discharged through the outlet of the extruder and pelletized by a pelletizer to obtain a modified polyalkylene oxide.

Synthesis of carboxyvinyl polymer

Production Example 2

Forty-five grams (0.625 mol) of acrylic acid, 0.27 g of pentaerythritol allyl ether, 150 g of n-hexane, and 0.081 g (0.00035 moles) of 2,2'-azobis methyl isobutyrate were placed in a 500-mL four-necked flask equipped with a stirrer, a thermometer, a nitrogen bubbler, and a condenser tube, to prepare a reaction mixture. After the reaction mixture was uniformly mixed by stirring, nitrogen gas was bubbled into the solution to remove oxygen present in the atmosphere of the reaction container, starting materials, and solvent. Subsequently, the reaction mixture was maintained at 60 to 65°C in a nitrogen atmosphere to allow a reaction to proceed for 4 hours. After completion of the reaction, the resulting slurry was heated to 90°C to distill off n-hexane. The residue was dried under reduced pressure at 110°C and 10 mmHg for 8 hours to obtain 42 g of a fine white powdery carboxy vinyl polymer powder.

Preparation of cosmetic

Example 1

Components 1 to 5 shown below were prepared as oil phase component A. Components 6 to 12 shown below were prepared as aqueous phase component A. The numerical value (%) shown after the name of each component

indicates the proportion of each component, based on the total mass of oil phase component A and aqueous phase component A.

First, an aqueous phase mixture was prepared using components 6 to 12 as aqueous phase component A and maintained at 60°C. On the other hand, while a mixture of components 1 to 3 for oil phase component A was maintained at 60°C, components 4 and 5 for oil phase component A were added. Then, while the resulting mixture was subjected to a dispersion treatment using a homomixer, the aqueous phase mixture maintained at 60°C was added. The resulting mixture was emulsified with a homomixer at a rotational speed of 10,000 rpm for 5 minutes to obtain an oil-in-water type cosmetic.

Oil phase component A

| | | |
|---|---|---|
| Component 1: | Liquid paraffin | 10% |
| Component 2: | Isopropyl palmitate | 10% |
| Component 3: | Ethylhexyl methoxycinnamate | 5% |
| Component 4: | Fine particulate titanium oxide (MT-100Z, Tayca Corporation) | 2% |
| Component 5: | Fine particulate zinc oxide (FINEX-30W, Sakai Chemical Industry Co., Ltd., particle size: 0.04 μm) | 2% |

Aqueous phase component A

| | | |
|---|---|---|
| Component 6: | Modified polyalkylene oxide (Production Example 1) | 0.3% |
| Component 7: | Polysorbate 60 | 3% |
| Component 8: | Glycerol | 5% |
| Component 9: | Propylene glycol | 5% |
| Component 10: | Ethanol | 3% |
| Component 11: | Phenoxyethanol | 0.5% |
| Component 12: | Purified water | Balance |

Component 3 is an ultraviolet absorber. Components 4 and 5 are (A) ultraviolet scattering agents. Component 7 is (B) a nonionic surfactant. Component 6 is (C) a modified polyalkylene oxide. Components 1 and 2 are (D) oily components. Component 12 is (E) water. The other components 8 to 11 are other components.

Example 2

An oil-in-water type cosmetic was prepared in the same manner as in Example 1 except that the proportion of fine particulate titanium oxide as component 4 was changed to 5%, the proportion of fine particulate zinc oxide as component 5 was changed to 10%, the proportion of modified polyalkylene oxide as component 6 was changed to 0.8%, and the balance amount of purified water as component 12 was changed to make the total mass of oil phase component A and aqueous phase component A 100 mass%.

Comparative Example 1

Components 1 to 6 shown below were prepared as oil phase component B. Components 7 to 12 shown below were prepared as aqueous phase component B. The numerical value (%) shown after the name of each component indicates the proportion of each component, based on the total mass of oil phase component B and aqueous phase component B. In Comparative Example 1, a modified polyalkylene oxide, which is component (C), was not used.

First, an aqueous phase mixture was prepared using components 7 to 13 as aqueous phase component B and maintained at 60°C. On the other hand, while a mixture of components 1 to 3 for oil phase component B was maintained at 60°C, components 4 to 6 for oil phase component B were added. Then, while the resulting mixture was subjected to a dispersion treatment using a homomixer, the aqueous phase mixture maintained at 60°C was added. The resulting mixture was emulsified with a homomixer at a rotational speed of 10,000 rpm for 5 minutes. However, the mixture was not uniformly dispersed and poorly emulsified, thus failing to obtain an oil-in-water type cosmetic.

Oil phase component B

| | | |
|---|---|---|
| Component 1: | Liquid paraffin | 10% |
| Component 2: | Isopropyl palmitate | 10% |
| Component 3: | Ethylhexyl methoxycinnamate | 5% |
| Component 4: | Carboxyvinyl polymer (Production Example 2) | 0.3% |
| Component 5: | Fine particulate titanium oxide (MT-100Z, Tayca Corporation) | 2% |
| Component 6: | Fine particulate zinc oxide (FINEX-30W, Sakai Chemical Industry Co., Ltd., particle size: 0.04 pm) | 2% |

Aqueous phase component B

| | | |
|---|---|---|
| Component 7: | 6 mass% aqueous sodium hydroxide solution | 1.6% |
| Component 8: | Polysorbate 60 | 3% |
| Component 9: | Glycerol | 5% |
| Component 10: | Propylene glycol | 5% |
| Component 11: | Ethanol | 3% |
| Component 12: | Phenoxyethanol | 0.5% |
| Component 13: | Purified water | Balance |

Evaluation of properties of cosmetics

The dispersion stability, viscosity, texture, and kinking of cosmetics were evaluated in the following manner.

Dispersion stability

Each cosmetic was allowed to stand at 50°C. The stability after one month was evaluated according to the following criteria.

A: The stably emulsified state was maintained;
B: A clear separation was observed; a stable emulsified state was not maintained.

Viscosity

Using "a BH type viscometer" produced by Toki Sangyo Co., Ltd., the viscosity was measured at a measurement temperature of 25°C at a rotational speed of 20 rpm.

Feel to the touch

A total of 10 testers consisting of 5 males and 5 females applied an appropriate amount of cosmetics to the back of their hands, spread them with fingers, and evaluated them in terms of refreshing feel and lightness in texture according to the following criteria:

A: Nine or more out of ten testers answered that the refreshing feel and lightness were excellent.
B: Eight out of ten testers answered that the refreshing feel and lightness were good.
C: One to seven out of ten testers answered that the refreshing feel and lightness were poor.

Kinking

A total of ten testers consisting of five males and five females applied and rubbed 0.5 g of each cosmetic on their forearms for 1 minute. Whether kinking or aggregates were formed was confirmed with the naked eye. The cosmetics were evaluated according to the following criteria.

A: Nine or more out of ten testers answered that no aggregates were formed.
B: Eight out of ten testers answered that a slight amount of aggregates were formed.
C: One to seven out of ten testers answered that a large amount of aggregates were formed.

Table 1

| Example/ Comparative Example | Dispersion stability | Viscosity (mPa•s) | Feel to the touch | Kinking |
|---|---|---|---|---|
| Example 1 | A | 3,000 | A | A |
| Example 2 | A | 25,000 | B | B |
| Comparative Example 1 | Failed to be produced | | | |

Industrial Applicability

The oil-in-water type cosmetic of the present invention is excellent in the dispersion stability of an ultraviolet scattering agent, can provide a refreshing feel and light sense of use with no stickiness to the skin, and also has a high ultraviolet-shielding effect. Therefore, the oil-in-water type cosmetic of the present invention is useful as a starting material for preparing various cosmetics, such as sunscreen cosmetics.

## Claims

1. An oil-in-water type cosmetic comprising:

   (A) an ultraviolet scattering agent;
   (B) a nonionic surfactant;
   (C) a modified polyalkylene oxide represented by the following formula (I):

   ( I )

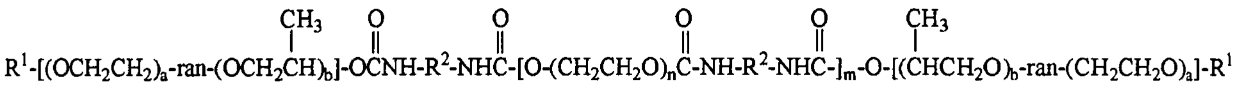

   wherein $R^1$ is a linear alkyl group having 15 to 24 carbon atoms, $R^2$ is a divalent organic residue derived from a diisocyanate compound, $[(OCH_2CH_2)_a\text{-ran-}(OCH_2CH(CH_3))_b]$ is a random copolymer of $OCH_2CH_2$ and $OCH_2CH(CH_3)$, which are constituent units of the copolymer, a is an integer of 5 to 10, b is an integer of 5 to 8, n is an integer of 130 to 680, and m is an integer of 1 to 4;
   (D) an oily component; and
   (E) water,

   the ultraviolet scattering agent comprising fine particulate titanium oxide having a mean particle diameter of 10 to 50 nm and/or fine particulate zinc oxide having a mean particle diameter of 10 to 50 nm.

2. The oil-in-water type cosmetic according to claim 1, wherein the ultraviolet scattering agent is titanium oxide and/or zinc oxide.

3. A sunscreen cosmetic comprising the oil-in-water type cosmetic according to claim 1 or 2.

## Patentansprüche

1. Ein Kosmetikum vom Öl-in-Wasser-Typ, umfassend:

   (A) ein Ultraviolett-streuendes Mittel;
   (B) ein nichtionisches oberflächenaktives Mittel;

(C) ein modifiziertes Polyalkylenoxid, dargestellt durch die folgende Formel (I):

( I )

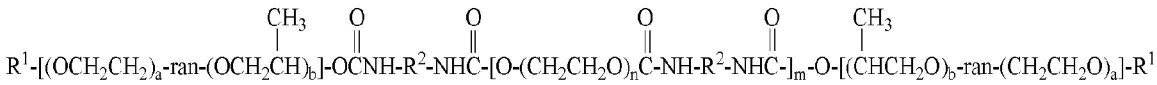

wobei $R^1$ eine lineare Alkylgruppe mit 15 bis 24 Kohlenstoffatomen ist, $R^2$ ein zweiwertiger organischer Rest ist, der von einer Diisocyanatverbindung abgeleitet ist, $[(OCH_2CH_2)_a$-ran-$(OCH_2CH(CH_3))_b]$ ein statistisches Copolymer von $OCH_2CH_2$ und $OCH_2CH(CH_3)$ ist, die konstituierende Einheiten des Copolymers sind, a eine ganze Zahl von 5 bis 10 ist, b eine ganze Zahl von 5 bis 8 ist, n eine ganze Zahl von 130 bis 680 ist und m eine ganze Zahl von 1 bis 4 ist;
(D) eine ölige Komponente; und
(E) Wasser,

wobei das Ultraviolett-streuende Mittel feinteiliges Titanoxid mit einem mittleren Teilchendurchmesser von 10 bis 50 nm und/oder feinteiliges Zinkoxid mit einem mittleren Teilchendurchmesser von 10 bis 50 nm umfasst.

**2.** Das Kosmetikum vom Öl-in-Wasser-Typ nach Anspruch 1, wobei das Ultraviolett-streuende Mittel Titanoxid und/oder Zinkoxid ist.

**3.** Ein Sonnenschutzkosmetikum, umfassend das Kosmetikum vom Öl-in-Wasser-Typ nach Anspruch 1 oder 2.

## Revendications

**1.** Cosmétique de type huile-dans-eau comprenant :

(A) un agent diffusant les ultraviolets ;
(B) un tensioactif non ionique ;
(C) un polyoxyde d'alkylène modifié représenté par la formule (I) suivante :

( I )

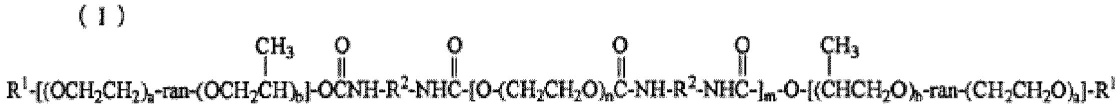

où $R^1$ est un groupe alkyle linéaire ayant 15 à 24 atomes de carbone, $R^2$ est un résidu organique divalent dérivé d'un composé diisocyanate, $[(OCH_2CH_2)_a$-ran-$(OCH_2CH(CH_3))_b]$ est un copolymère aléatoire de $OCH_2CH_2$ et $OCH_2CH(CH_3)$, qui sont des motifs constitutifs du copolymère, a est un entier de 5 à 10, b est un entier de 5 à 8, n est un entier de 130 à 680, et m est un entier de 1 à 4 ;
(D) un composant huileux ; et
(E) de l'eau,

l'agent diffusant les ultraviolets comprenant de l'oxyde de titane en particules fines ayant un diamètre moyen de particule de 10 à 50 nm et/ou de l'oxyde de zinc en particules fines ayant un diamètre moyen de particule de 10 à 50 nm.

**2.** Cosmétique de type huile-dans-eau selon la revendication 1, dans lequel l'agent diffusant les ultraviolets est l'oxyde de titane et/ou l'oxyde de zinc.

**3.** Cosmétique écran solaire comprenant le cosmétique de type huile-dans-eau selon la revendication 1 ou 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2012251603 A
- JP 2002326906 A
- JP 2011046670 A
- JP 2013091625 A